# EUROPEAN PATENT SPECIFICATION

(11) **EP 3 682 923 B1**
(45) Date of publication and mention of the grant of the patent: **15.12.2021**
(21) Application number: 19151965.1
(22) Date of filing: 15.01.2019
(51) Int. Cl.: A61M 15/00

(54) **MEDICATION INHALER FOR AEROSOL PULMONARY DELIVERY WITH VISUAL FEEDBACK SYSTEM**
MEDIKAMENTENINHALATOR ZUR AEROSOLPULMONALEN VERABREICHUNG MIT VISUELLEM FEEDBACK-SYSTEM
INHALATEUR DE MÉDICAMENT POUR ADMINISTRATION PULMONAIRE D'AÉROSOL COMPORTANT UN SYSTÈME DE RÉTROACTION VISUELLE

(43) Date of publication of application: 22.07.2020
(73) Proprietor: Air Liquide Medical Systems S.R.L., 20158 Milano (IT)
(72) Inventor: RUOCCO, Alessandra, 25073 Bovezzo (IT); ALBERICI, Luca, 25073 Bovezzo (IT); SANDONI, Giuseppe, 25073 Bovezzo (IT)
(74) Representative: Air Liquide

(56) References cited:
- EP-A2- 0 769 303
- WO-A1-90/09203
- WO-A1-99/53982
- WO-A1-2012/061866
- WO-A1-2017/163211
- US-A- 5 522 380
- US-A- 6 039 042
- US-A1- 2012 318 261
- US-B2- 7 201 164

## Description

The present invention relates to a medication inhaler useable with a metered dose inhaler (MDI) for delivering a respiratory gaseous therapy, especially metered or pre-metered aerosolized doses of a medicinal product mixed with and transported by a carrier gas, such as air, to the human respiratory system of a patient in need thereof, especially of pediatric patients or the like, said medication inhaler comprising a visual feedback system allowing to immediately see whether a pediatric patient breathes through the medication inhaler.

Aerosol drug delivery (ADD) devices are used for delivering various respiratory gaseous therapies, such as inhalable aerosols containing micro-particles or droplets of medication (i.e. a drug) to the lungs of a patient.

An aerosol drug delivery (ADD) device usually comprises a medication inhaler fluidly coupled to a metered dose inhaler (MDI), typically a canister containing a medication and a pressurized gas for dispersing the medication.

The medication inhaler generally comprises an elongated hollow holding chamber comprising an inlet opening into which the MDI is plugged, and an oulet opening in fluid communication with a respiratory interface, such as a respiratory mask or mouthpiece. The medication/gas mixture is delivered by the MDI into the hollow holding chamber of the medication inhaler, where it is mixed with a carrier gas, typically air, thereby generating a gaseous therapy containing micro-particles or droplets of medication, that is subsequently inhaled by a patient in need thereof.

A diaphragm valve, such as a one-way valve, is generally arranged in the medication inhaler, typically adjacent to the respiratory interface, and designed/configured to open only when the patient inhales, for allowing a passage of medication from the hollow holding chamber of the medication inhaler to the lungs of the patient.

ADD devices or assemblies comprising medication inhalers coupled to MDIs are disclosed by document EP-A-2014325 and WO-A-03/097142.

Generally, several breaths of the patient are required for a full administration of the prescribed or desired drug dosage, i.e. so that the respiratory gaseous therapy is efficient.

In other words, medication inhalers are efficient only if the patient accepts and follows his/her inhalation therapy.

However, it has been noted in practice that it is not or rarely the case for a given population of patients, namely "pediatric patients", such as children, toddlers, babies or the like, as they are often either scared by medication inhalers and/or unable to stay calm while a medication inhaler is applied on their face. Some of them even totally refuse to inhale the aerosol.

As a consequence, it is often difficult to know whether a paediatric patient, i.e. infant, baby, toddler or the like, has inhaled at least a part of the aerosolized medication delivered by a medication inhaler and thus has been at least partially treated.

This is obviously an issue as it may lead to a lack of efficiency of the inhalation therapy if no or only a very low amount of medication has been inhaled by the pediatric patient.

Examples of aerosol medication delivery devices are given by US7201164, WO99/53982, WO2017/163211, US6039042, WO2012/061866, US2012/318261 and WO90/09203.

A problem is hence to provide an improved medication inhaler allowing a physician, a nurse or any other person (e.g. parents), to immediately know whether a patient, especially a paediatric patient, is correctly inhaling a gaseous therapy while it is delivered by a medication inhaler.

A solution according to the present invention is a medication inhaler for delivering a gaseous therapy to a patient, especially a paediatric patient, comprising a housing, a top cover fixed to the housing, and an inner compartment arranged between said housing and said top cover, and wherein:
- the housing comprises a hollow chamber with an exit orifice, and the top cover comprises an outlet, said exit orifice of the hollow chamber and said outlet of the top cover being in fluid communication with the inner compartment,
- a venting channel comprising an entry port and a venting port, is in fluid communication with the inner compartment via the entry port and further with the atmosphere via the venting port, and
- a one-way valve is arranged in the inner compartment and configured for allowing gas to circulate :
   i) from the hollow chamber of the housing to the outlet of the top cover, when the patient inhales gas, and
   ii) from the outlet of the top cover to the venting channel, when the patient exhales gas,
   wherein the venting channel comprises a mobile element that moves into said venting channel in the direction of the venting port, said mobile element being retained in the area of the venting port and visible from the outside through said venting port, when the patient exhales gas, especially CO₂-enriched gas, through the outlet of the top cover, thereby providing a visual feedback system allowing to immediately know from the outside, whether said patient is breathing gas,
   characterized in that:
   - the mobile element has a spherical or cylindrical shape,
   - at least a portion of the venting channel and the mobile element are arranged into a removable cassette having an elongated arcuated shape, and
   - the removable cassette further comprises an inner passage with an inlet and an outlet, said inner passage forming at least a part of the venting channel.

According to the invention, said mobile element moving "back and forth" into the venting channel, when the paediatric patient breathes gas, provides a visual feedback system allowing to immediately know from the outside, whether said paediatric patient is breathing gas. This provides a very useful information to the caregiver (e.g. nurse, physician, parents...).

In accordance with the present invention, the "gaseous therapy" can comprise at least a therapeutically-effective compound, such as a medication or drug product (e.g. dropelets, powder, gaseous compound, aerosol...) mixed with and/or transported by a carrier gas, such as air.

The medication inhaler for aerosol therapy, i.e. the aerosol inhalation device, according to the present invention can comprise one or several of the following additional features :
- the one-way valve comprises a flexible peripheral skirt that flexes toward housing, when the patient exhales gas through the outlet of the top cover, thereby allowing expired gas to enter into venting channel via entry port.
- the one-way valve is at least partially flexible.
- the mobile element is a ball or the like, preferably a light little ball or the like.
- the top cover comprises :
   - a sleeve portion forming a sleeve around a top end of the housing, when the top cover is attached to the housing, and
   - a roof portion secured to the sleeve portion, and comprising the outlet of the top cover.
- the inner chamber is delimited by the inner walls of the sleeve portion and of the roof portion of the top cover, and an outer end wall of the housing.
- the outer end wall of the housing forms the bottom wall of the inner chamber.
- the sleeve portion of the top cover comprises a window facing and in fluid communication with the venting port of the venting channel, when the top cover is attached to the housing.
- the one-way valve faces the exit orifice of the housing.
- the one-way valve comprises a peripheral annular border is sandwiched and squeezed between the top cap and the housing, thereby forming a gas seal.
- the mobile element has a diameter of less than 10 mm, preferably of about 5 mm.
- the mobile element has a weight of less than 1 g, preferably less than 0.5 g, more preferably less than 0.1 g, for instance of about 0.08 g.
- the mobile element is made from polymer material, preferably plastic, avantageously polyamide (PA).
- the mobile element is a ball (i.e. sphere) made of polyamide (PA), such as Nylon^{™} or the like.
- the mobile element can be colored for being better visible from the outside, for instance it can be red, orange, white, yellow etc...
- the top cover is detachably fixed to the upper end (i.e. top end) of the housing.
- the housing further comprises a bottom cover.
- the bottom cover is fixed to the bottom end of the housing.
- the bottom cover is detachably fixed to the bottom end of the housing, i.e. removable.
- the bottom cover comprises a bottom inlet for plugging an aerosol drug delivery (ADD) device thereto, such as a MDI (metered dose inhaler).
- the top cover has a generally cup-like shape.
- the bottom cover and/or the top cover are detachably fixed to the housing by a snap-fit connection system, a screw connection system or any other kind of locking mechanism.
- the housing, the bottom cover and the top cover are made of polymer material, such as plastic or the like.
- the one-way valve is a duck-beak valve.
- the one-way valve is sandwiched between the housing and the top cover.
- the one-way valve is a duck-beak valve made of a flexible material.
- the one-way valve is made of a polymeric material, such as silicone, elastomer, rubber or the like.
- the one-way valve is molded in one-piece.
- the one-way valve comprises a flexible peripheral collar or skirt.
- the peripheral collar or skirt of the one-way valve is traversed by several holes, preferably at least 3 holes, cooperating with several pins, preferably at least 3 pins, carried by the housing for well-positioning and securing the one-way valve.
- the pins of the housing are lodged into correspoding holes of the collar or skirt of the one-way valve, when the one-way valve is positioned/arranged on the housing, typically sandwiched between the housing and the top cover, for thereby maintaining the one-way valve in a defined and fixed position on the housing, i.e. between top cover and housing.
- the one-way valve comprises at least one slot for allowing the aerosolized flow passing therethrough, when the patients inhales the gaseous therapy from the hollow inner chamber of the housing.
- the housing has a general tubular shape, such as a tronconical or cylindrical shape.
- the housing is elongated.
- a respiratory interface, such as a respiratory mask or mouthpiece, is fixed to the top cover, preferably detachably fixed thereto.
- the outlet of the top cover is in fluid communication with the respiratory interface, such as a respiratory mask or mouthpiece.
- the bottom intlet of the bottom cover constitutes a central opening for plugging an ADD device thereto.
- the bottom intlet of the bottom cover is configured and sized for receiving and holding an ADD device.

The present invention also concerns an aerosoltherapy assembly comprising a medication inhaler as described, and an aerosol drug delivery (ADD) device plugged into the bottom inlet of the bottom cover of said medication inhaler.

The present invention will be explained in more details in the following illustrative description of an embodiment of a medication inhaler for aerosoltherapy, which is made in references to the accompanying drawings among them:
- Figure 1 represents a side view of an embodiment of a medication inhaler according to the present invention comprising a top cap secured to a housing,
- Figure 2 represent a side view of the upper part of the medication inhaler of Figure 1,
- Figure 3 shows the one-way valve arranged on the housing of the medication inhaler of Figures 1 and 2,
- Figures 4 and 5 show how the one-way valve of Figure 3 works during inspiration and expiration phases of the patient,
- Figure 6 represents an enlarged (partial) view of the upper part of the medication inhaler of Figure 1 showing the venting channel and the mobile element arranged in it,
- Figure 7 represents an embodiment of a removable cassette comprising the venting channel and the mobile element of Figure 6,
- Figure 8 represents a cross-sectional view of the upper part of the housing of the medication inhaler of Figure 3,
- Figure 9 represents an enlarged (partial) view of the upper part of the medication inhaler of Figure 1 shown without the one-way valve,
- Figure 10 shows the mobile element when located into the venting channel in the area of the venting port,
- Figure 11 represents a cross-sectional view of the upper part of the of the medication inhaler of Figure 1,
- Figures 12 and 13 are side vews of the upper part of the of the medication inhaler of Figure 1 comprising a cassette as shown in Figure 7, and
- Figure 14 is a side view of the upper part of the medication inhaler of Figure 1 comprising a cassette as shown in Figure 7 and a duck-beak valve.

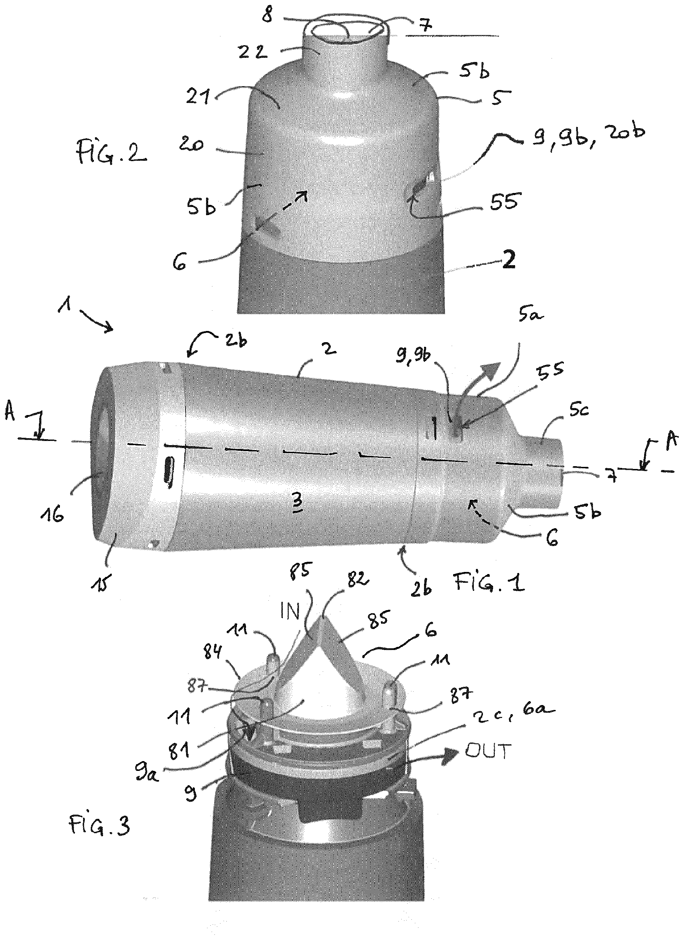

Figure 1 represents a side view of an embodiment of a medication inhaler 1 according to the present invention, also called a "spacer", that is suitable for providing a gaseous therapy to a patient, i.e. an aerosol drug pulmonary delivery.

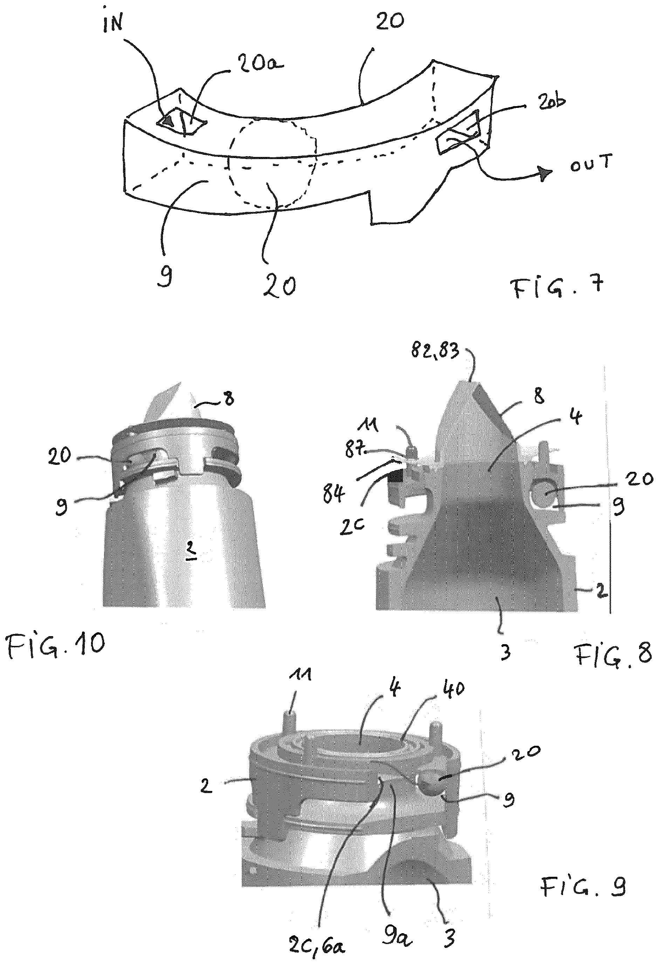

The medication inhaler 1 comprises a housing 2 having an ellongated tubular shape having a longitudinal axis A-A, preferably a tronconical or cylindrical shape. The housing 2 comprises an inner hollow chamber 3 (cf. Fig. 8) for containing the gaseous therapy (i.e. gaseous drug or the like) to be provided to the patient. The housing 2 further comprises an exit orifice 4 (cf. Fig. 8) fluidly communicating with the hollow chamber 3 for allowing the gaseous therapy to leave the hollow chamber 3 and circulates in the direction of the patient's airways, while passing through the one-way valve 8 and the outlet 7 of the top cover 5, as below explained.

The housing 2 comprises two opposite ends, namely a top end 2a and a bottom end 2b. A bottom cover 15 is detachably fixed to the bottom end 2b, whereas a top cover 5 is detachable fixed to the top end 2a. The bottom cover 15 and/or the top cover 5 are attached to the housing 2 by a snap-fit connection system, a screw connection system or any other suitable locking mechanism or the like.

Preferably, the housing 2, the bottom cover 16 and the top cover 15 are made from polymer material, such as plastic, or the like.

The bottom cover 15 secured to housing 2 comprises a bottom intlet 16 for plugging an aerosol drug delivery (ADD) device thereto, i.e. the bottom intlet 16 constitutes a central opening fluidly communicating with inner hollow chamber 3 of housing 2. Preferably, intlet 16 is configured and sized, i.e. structured and/or designed, for receiving and holding an ADD device that is plugged therein.

Further, top cover 5 comprises an outlet 7 in fluid communication with the hollow chamber 3 of the housing 2, via the exit orifice 4 of said housing 2. Outlet 7 is further in fluid communication with a respiratory interface (not shown), such as a respiratory mask or mouthpiece, that is detachably secured to the top cover 5, and used to administering the gaseous therapy to the patient.

The outlet 7 is arranged in the roof portion 5b, also called roof 21, of top cover 5, preferably outlet 7 is carried by a hollow neck 22 or the like, that can be located at the center of the roof portion 5b of top cover 5.

Preferably, the outlet 7 of top cover 5, the bottom intlet 16 of bottom cover 15 and the the exit orifice 4 of housing 2 are co-axially arranged, i.e. on axis A-A of the housing 2 as shown in Figure 1.

In the embodiment of Figures 1 and 2, top cover 5 has a generally cup-like form. It comprises a sleeve portion 20 (i.e. tubullar) having a generally-cylindrical or tronconical shape and terminated/closed, at its top end, by roof 21 comprising the hollow neck 22 terminated by outlet 7.

Top cover 5, namely sleeve portion 20 and roof 21, defines with the top end 2a of housing 2, an inner compartment 6.

When top cover 5 is secured to housing 2, as represented in Figures 1 and 2, inner compartment 6 delimited by the inner walls of top cover 5 and outer upper end wall 2c of housing 2, is in fluid communication with:
- on the one hand, with a respiratory interface (i.e. mask or the like) via the lumen of the hollow neck 22 and outlet 7, and
- on the other hand, with the hollow chamber 3 of housing 2, via exit orifice 4.

In other words, the outer upper end wall 2c of housing 2 constitues at least a part of the bottom wall 6a of the inner compartment 6.

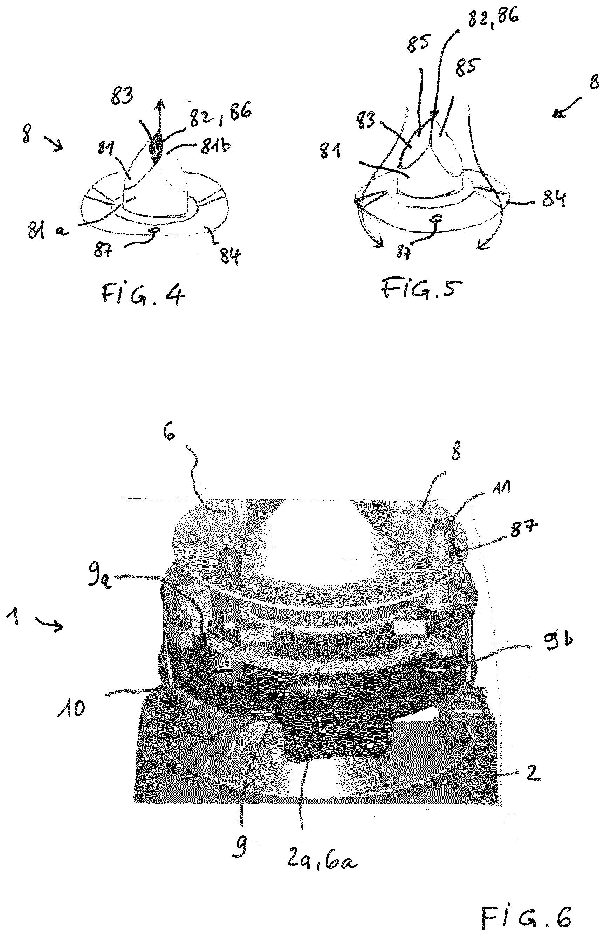

For controlling the gas flows in inner chamber 6, a one-way valve 8, preferably a duck beak valve as shown in Figures 4 and 5, is arranged between top cap 5 and housing 2, as shown in Figures 3 and 8. One-way valve 8 faces the exit orifice 4 of housing 2.

More precisely, one-way valve 8 is designed and arranged for allowing gas to circulate :
i) from hollow chamber 3 of housing 2 toward outlet 7 of top cover 5, i.e. through inner compartment 6, when the patient inhales gas (i.e. a gaseous medication) via the neck 22 carrying the outlet 7 and the respiratory interface secured to neck 22, as shown in Figure 4, and
ii) from outlet 7 of top cover 5 back to inner compartment 6, when the patient exhales gas, i.e. CO₂-enriched gases coming from the lungs of the patient, into the respiratory interface fluidly connected to outlet 7, especially to the neck 22 of top cover 5, as shown in Figure 5.

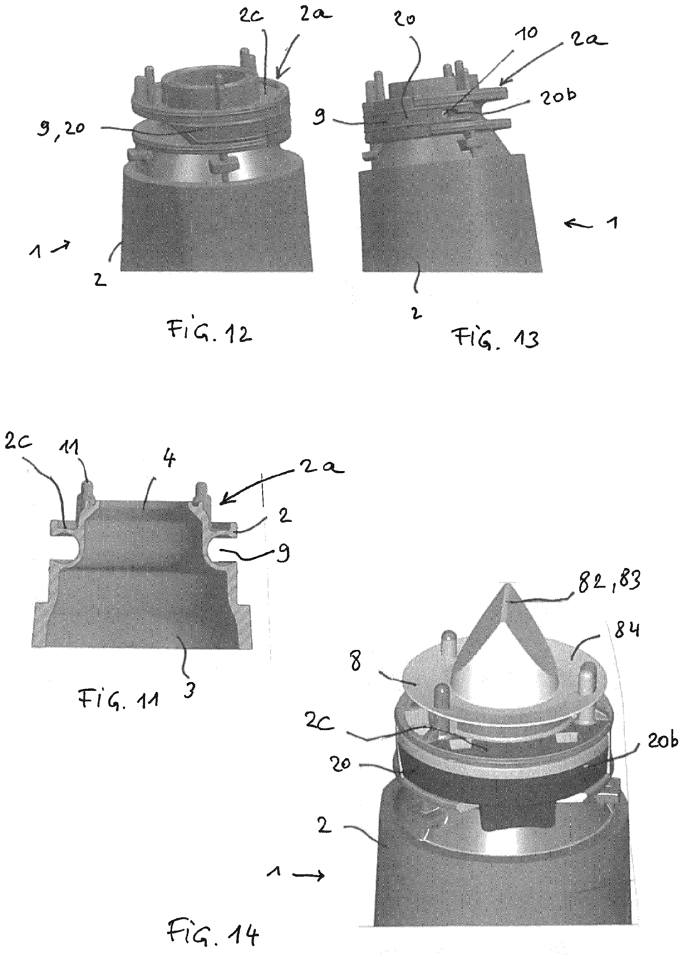

In other words, one-way valve 8, avantageously a duck beak valve as shown in Figures 4, 5 and 14, opens to allow a gaseous flow, e.g. an aerosolized medication generated into hollow chamber 3 of housing 2, to reach the patient's airways, during the inhalation phases of the patient, whereas it closes for prohibiting CO₂-enriched gases expired by the patient to flow back into hollow chamber 3 of housing 2, during the exhalation (i.e. expiration) phases of the patient.

An embodiment of a duck beak valve useable as one-way valve 8 is shown in Figures 4, 5 and 14. It comprises a hollow body 81 comprising a cylindrical portion 81a and a duck beak shape portion 81b defining a beak outlet 83 comprising a central slot 82 for controlling the gas flow. The slot 82 is located at the apex or crest 86 of two angled surfaces 85 projecting upwardly and that meet at said crest 86.

Slot 82 allows the aerosolized flow exiting the ellongated hollow chamber 3 of housing 2, to pass therethrough, in the direction of the outlet 7 of the top cover 5 so as to reach the user's airways and to treat him/her. In other embodiments, the duck beak valve useable as one-way valve 8 can comprise several slots 82, such as 2 slots forming together a "+", an "x" or any other suitable shape.

A peripheral annular border 84 forming a flexible peripheral skirt is attached to and surrounding the cylindrical portion 81a of the hollow body 81 of the valve 8. When sandwiched between the top cap 5 and the housing 2, the peripheral annular border 84 or flexible peripheral skirt is squeezed between walls or structures of top cap 5 and housing 2, such as the annular rim or border 40 located around exit orifice 4 (cf. Fig. 9), thereby forming a gas seal that forces the gas to pass through slot 82, during the inspiratory phases of the patient, and, in contrast, to prohibit CO₂-enriched gas to leak between top cover and housing, and go back into the inner chamber 3 of the housing 2, during the expiratory phases of the patient.

For well-positioning and securing the one-way valve 8 between top cap 5 and housing 2, several holes 87, preferably at least 3 holes, are arranged in the peripheral annular border 84 of the one-way valve 8, that cooperate with several pins 11, picots or the like arranged in the housing 2, for instance arranged in the outer upper end wall 2c of housing 2 that constitues also the bottom wall 6a of inner compartment 6. Pins 11 are lodged into holes 87 of flexible skirt 84 of the one-way valve 8, when the one-way valve 8 is positioned and secured on the housing 2, i.e. when sandwiched between the housing 2 and the top cover 5, for thereby maintaining the one-way valve 8 in a defined and fixed position on the housing 2, as shown in Figures 3 and 6.

The duck beak valve constituting one-way valve 8 is made from a flexible/resilient material, such as silicone, elastomer, rubber or the like, preferably molded in one-piece.

The duck beak valve works as follows :
- during the inhalation phases of the patient (Fig. 4), the drug-containing gas passes through the duck beak valve constituting a one-way valve 8, namely it flows through the lumen of the hollow body 81 and exits through the slot 82 of the beak outlet 83 as said slot 82 is open by the gas pressure/flowrate, and
- during the expiration phases of the patient (Fig. 5), the gases expired by the patient, namely CO₂-enriched gases, cannot pass through the duck beak valve due to its particular shape, as the slot 82 of the beak outlet 83 is closed by the action of the pressure/flowrate of the gases expired by the patient that acts on the angled surfaces 85. At the same time, expired gases create on overpressure into inner compartment 6 of top cover 5 that bends and deforms one or several portions of the peripheral annular border 84 forming the flexible peripheral skirt of the duck beak valve, thereby creating gas passages (i.e. leak passages) for the expired gases, allowing them to escape the inner compartment 6 and be vented to the atmosphere, through a venting channel 9 arranged in the body of the housing 2. In other words, CO₂-enriched gases expired by the patient are recovered by inner compartment 6 and immediatey evacuated by venting channel 9 that is fluidly communicating, on the one hand, with inner compartment 6 and, on the other hand, with the atmosphere.

More precisely, venting channel 9 is arranged in the housing 2 of the medication inhaler 1 as shown in Figures 6 and 8. It comprises an entry port 9a and a venting port 9b.

Depending on the embodiment, venting channel 9 can be directly or indirectly arranged in the body of housing 2. In particular, the venting channel 9 can be arranged in an independant element that is subsequently secured to the body of housing 2, such as the removable cassette 20 detailed hereafter in connexion with Fig. 7.

The entry port 9a of venting channel 9 is in fluid communication with the inner compartment 6 of the top cover 5, for example entry port 9a can be arranged directly in the outer upper end wall 2c of housing 2 that constitues also the bottom wall 6a of inner compartment 6 as shown in Figure 9.

Further, venting port 9b is in fluid communication with the atmosphere for allowing CO₂-enriched gas to be vented to the atmosphere.

Venting channel 9 has preferably an elongated arcuated shape as shown in Figure 6.

According to one embodiment shown in Figure 7, the venting channel 9 is indirectly arranged in housing 2 as it is at least partially formed into an elongated arcuated hollow element forming a removable cassette 20 that is fixed to the housing 2, for example plugged into it, as shown in Figures 3, 6, and 12-14.

The removable cassette 20 shown in Figure 7 comprises an inner passage terminated by orifices, i.e. an inlet 20a located at one end and an outlet 20b at the other end, and forming at least a part of the venting channel 9.

Said removable cassette 20 is secured into housing 2 so that its inlet 20a is positioned so as to face the entry port 9a arranged in the bottom 6a of inner compartment 6. In other words, inlet 20a of removable cassette 20 and entry port 9a are in fluid communication so that gas passing through entry port 9a (in the direction of venting port 9b) can enter into the lumen of said removable cassette 20 that constitutes, in this embodiment, at least a part of the venting channel 9.

At the other end, venting port 9b can be either the outlet 20b itself of the removable cassette 20, or another orifice, preferably facing the outlet 20b of removable cassette 20 or fluidly communicating with it, depending on the embodiment.

Furthermore, in the embodiment shown in Figures 1 and 2, top cover 5 also comprises a window 15 forming a large opening that is positionned in front of, i.e. facing, the venting port 9b, when the top cover 5 is secured onto housing 2, for instance by a screw, snap-fit or any other connection system. However, window 15 can have various shapes, such as a notch.

Further, in other embodiments, the sleeve portion 5a of top cover 5 can be shorter and not recover the venting port 9b. In this case, a window 15, notch or the like is not required.

As above explained, a lot of pediatric patients, such as babies, toddlers or young infants, are either scared by medication inhalers and/or unable to stay calm while a medication inhaler is applied on their face. As a consequence, they either totally refuse to inhale the aerosol or, at best, inhale only a part of the medication. This may result in a lack of efficiency of the inhalation therapy, depending on the amount of aerosolized medication inhaled by the paediatric patient.

In other words, it is often difficult to know whether a paediatric patient, i.e. infant, baby, toddler or the like, has inhaled at least a part of the aerosolized medication delivered by a medication inhaler and thus has been at least partially treated.

According to the present invention, for assisting the medical staff or the like in its assessment of the amount of medication inhaled by a paediatric patient, a visual feedback system has been incorpoated into the medication inhaler 1 that provides an immediate information on the accomplishment of the respiratory acts.

More precisely, the visual feedback system according to the present invention comprises, i.e. a visual indicator, namely a mobile element 10 arranged into the venting channel 9, that can have a sphere, cylinder, ovoid or any other suitable shape.

According to a preferred embodiment, the mobile element 10 is a little ball having a diameter that is sligthly less than the inner cross-dimension, namely the inner diameter or the like, of the lumen of the venting channel 9, for intance the inner cross-dimension of the inner passage traversing the removable cassette 20 of Figure 7. For instance, the mobile element 10 is a light little ball having a diameter of between 3 and 10 mm, preferably of between 4 and 7 mm, and/or a weight of less than 0.5 g, preferably less than 0.2 g, for instance of about 0.1 g, e.g. 0.08 g.

The mobile element 10 is preferably made from a light material, such as plastic or the like, such as polyamide (PA), for instance Nylon^{™}.

The mobile element 10 can freely move, i.e. is mobile, into the venting channel 9 in the direction of the venting port 9b, when the patient exhales a CO₂-enriched gas through the outlet 7 of the top cover 5 that successively flexes the flexible skirt of one-way valve 8, i.e. a duck beak valve, escapes the inner compartment 6 via entry port 9a of venting channel 9, circulates into venting channel 9 toward venting port 9b and is subsequantly evacuated to the atmosphere.

Thus, when the patient exhales gases, said gases push against the mobile element 10, i.e. little ball or the like, thereby forcing it to move toward the venting port 9b. Preferably, the size of venting port 9b, e.g. its diameter or the like, is chosen to be less than the size of the mobile element 10, e.g. its diameter in the case of a ball, so that said mobile element 10 is kept into venting channel 9 and not allowed to escape.

In other words, the light mobile element 10 abuts against the inner walls of the venting channel 9 in the area of the venting port 9b but is retained in it, while being visible from the outside through said venting port 9b, as shown in Figure 10.

This allows the medical staff or members of patient's family, to immediately see said mobile element 10, from the outside, and ascertain whether or not the patient breathes in the inhalation inhaler 1, especially whether or not the patient has expired CO₂-enriched gases through the venting channel 9. Indeed, if it is the case, the mobile element 10 will be pushed by the gas flow toward the venting port 9b and will show up in the area of the venting channel 9 located immediately behind the venting port 9b and be visible from the outside.

Thanks to the invention, the medical staff can also count the number of performed breaths, especially expiration phases of the patient, as they correspond to the number of times that the mobile element 10 travelling into the venting channel 9, has appeared in the area of the venting port 9b, over a given period of time, while the medication is delivered, thanks to the inhalation inhaler 1. This allows confirmation that the paediatric patient is breathing in the inhalation inhaler 1.

## Claims

1. Medication inhaler (1) for delivering a gaseous therapy to a patient comprising a housing (2), a top cover (5) fixed to the housing (2) and an inner compartment (6) arranged between said housing (2) and said top cover (5), and wherein:
- the housing (2) comprises a hollow chamber (3) with an exit orifice (4), and the top cover (5) comprises an outlet (7), said exit orifice (4) of the hollow chamber (3) and said outlet (7) of the top cover (5) being in fluid communication with the inner compartment (6),
- a venting channel (9) comprising an entry port (9a) and a venting port (9b), is in fluid communication with the inner compartment (6) via the entry port (9a) and further with the atmosphere via the venting port (9b), and
- a one-way valve (8) is arranged in the inner compartment (6) and configured for allowing gas to circulate :
i) from the hollow chamber (3) of the housing (2) to the outlet (7) of the top cover (5), when the patient inhales gas, and
ii) from the outlet (7) of the top cover (5) to the venting channel (9), when the patient exhales gas,
wherein the venting channel (9) comprises a mobile element (10) that moves into said venting channel (9) in the direction of the venting port (9b), said mobile element (10) being retained in the area of the venting port (9b) and visible from the outside through said venting port (9b), when the patient exhales gas through the outlet (7) of the top cover (5), thereby providing a visual feedback system allowing to immediately know from the outside, whether said patient is breathing gas,
**characterized in that**:
- the mobile element (10) has a spherical or cylindrical shape,
- at least a portion of the venting channel (9) and the mobile element (10) are arranged into a removable cassette (20) having an elongated arcuated shape, and
- the removable cassette (20) further comprises an inner passage with an inlet (20a) and an outlet (20b), said inner passage forming at least a part of the venting channel (9).

2. Medication inhaler (1) according to the preceding claim, **characterized in that** the one-way valve (8) is a duck-beak valve, preferably at least partially flexible.

3. Medication inhaler (1) according to any one of the preceeding claims, **characterized in that** the one-way valve (8) comprises a flexible peripheral skirt (84) that flexes toward housing (2), when the patient exhales gas through the outlet (7) of the top cover (5), thereby allowing expired gas to enter into venting channel (9) via entry port (9a).

4. Medication inhaler (1) according to any one of the preceeding claims, **characterized in that** the mobile element (10) is a ball.

5. Medication inhaler (1) according to Claim 1, **characterized in that**:
- the inlet (20a) of the removable cassette (20) and the entry port (9a) of the venting channel (9) are in fluid communication, and
- the outlet (20b) of the removable cassette (20) either constitutes the venting port (9b), or faces and is in fluid communication with the venting port (9b).

6. Medication inhaler (1) according to any one of the preceeding claims, **characterized in that** the top cover (5) comprises :
- a sleeve portion (5a) forming a sleeve around a top end (2b) of the housing (2), when the top cover (5) is attached to the housing (2),
- a roof portion (5b) secured to the sleeve portion (5a), and comprising the outlet (7) of the top cover (5).

7. Medication inhaler (1) according to any one of the preceeding claims, **characterized in that** the inner chamber (6) is delimited by the inner walls of the sleeve portion (5a) and of the roof portion (5b) of the top cover (5), and an outer end wall (2c) of the housing (2), preferably the outer end wall (2c) of the housing (2) forms the bottom wall (6a) of the inner chamber (6).

8. Medication inhaler (1) according to claim 6, **characterized in that** the sleeve portion (5a) of the top cover (5) comprises a window (55) facing and in fluid communication with the venting port (9b; 20b) of the venting channel (9), when the top cover (5) is attached to the housing (2).

9. Medication inhaler (1) according to any one of the preceeding claims, **characterized in that** the one-way valve (10) faces the exit orifice (4) of the housing (2) and/or comprises a peripheral annular border (84) is sandwiched and squeezed between the top cap (5) and the housing (2), thereby forming a gas seal.

10. Medication inhaler (1) according to any one of the preceeding claims, **characterized in that** the venting channel (9) has an elongated arcuated shape.

11. Medication inhaler (1) according to any one of the preceeding claims, **characterized in that** the mobile element (10) has a diameter of less than 10 mm, preferably of about 5 mm.

12. Medication inhaler (1) according to any one of the preceeding claims, **characterized in that** the mobile element (10) is made from polymer material, preferably polyamide.

13. Medication inhaler (1) according to any one of the preceeding claims, **characterized in that** the housing (2) further comprises a bottom cover (15) comprising a bottom inlet (16) configured for plugging an aerosol drug delivery (ADD) device thereto.

14. Medication inhaler (1) according to any one of the preceeding claims, **characterized in that** the mobile element (10) has a weight of less 1 g, preferably less than 0,5 g.

15. Aerosoltherapy assembly comprising a medication inhaler (1) according to any one of the preceeding claims and an aerosol drug delivery (ADD) device plugged into the bottom inlet (16) of the bottom cover (15) of said medication inhaler (1).

## Patentansprüche

1. Medikamentinhalator (1) zur Zufuhr einer gasförmigen Therapie an einen Patienten, umfassend ein Gehäuse (2), eine obere Abdeckung (5), die an dem Gehäuse (2) befestigt ist, und ein inneres Fach (6), das zwischen dem Gehäuse (2) und der oberen Abdeckung (5) angeordnet ist, und wobei:
- das Gehäuse (2) eine hohle Kammer (3) mit einer Austrittsöffnung (4) umfasst und die obere Abdeckung (5) einen Auslass (7) umfasst, wobei die Austrittsöffnung (4) der hohlen Kammer (3) und der Auslass (7) der oberen Abdeckung (5) in Fluidverbindung mit dem inneren Fach (6) stehen,
- ein Entlüftungskanal (9), der einen Eintritts-Port (9a) und einen Entlüftungs-Port (9b) umfasst, über den Eintritts-Port (9a) in Fluidverbindung mit dem inneren Fach (6) ist und ferner über den Entlüftungs-Port (9b) mit der Atmosphäre und
- ein Einwegeventil (8) in dem inneren Fach (6) angeordnet und dazu ausgestaltet ist, das Zirkulieren von Gas:
i) aus der hohlen Kammer (3) des Gehäuses (2) zu dem Auslass (7) der oberen Abdeckung (5) zu gestatten, wenn der Patient Gas inhaliert, und
ii) aus dem Auslass (7) der oberen Abdeckung (5) zu dem Entlüftungskanal (9) zu gestatten, wenn der Patient Gas ausatmet,
wobei der Entlüftungskanal (9) ein bewegliches Element (10) umfasst, das sich in der Richtung des Entlüftungs-Ports (9b) in den Entlüftungskanal (9) bewegt, wobei das bewegliche Element (10) in dem Bereich des Entlüftungs-Ports (9b) gehalten wird und von außerhalb durch den Entlüftungs-Port (9b) sichtbar ist, wenn der Patient Gas durch den Auslass (7) der oberen Abdeckung (5) ausatmet, wodurch ein visuelles Rückmeldesystem bereitgestellt wird, dank dessen sofort von außen feststellbar ist, ob der Patient Gas einatmet,
**dadurch gekennzeichnet, dass**:
- das bewegliche Element (10) eine kugelförmige oder zylindrische Form hat,
- mindestens ein Abschnitt des Entlüftungskanals (9) und das bewegliche Element (10) in einer entfernbaren Kassette angeordnet sind, die eine längliche, bogenförmige Form hat,
und
- die entfernbare Kassette (20) ferner einen inneren Durchgang mit einem Einlass (20a) und einem Auslass (20b) umfasst, wobei der innere Durchgang mindestens einen Teil des Entlüftungskanals (9) bildet.

2. Medikamentinhalator (1) nach dem vorhergehenden Anspruch, **dadurch gekennzeichnet, dass** das Einwegeventil (8) ein vorzugsweise mindestens teilweise flexibles Entenschnabelventil ist.

3. Medikamentinhalator (1) nach einem der vorhergehenden Ansprüche, **dadurch gekennzeichnet, dass** das Einwegeventil (8) ein flexibles Umfangshemd (84) umfasst, das sich zum Gehäuse (2) hin biegt, wenn der Patient Gas durch den Auslass (7) der oberen Abdeckung (5) ausatmet, wodurch verbrauchtes Gas über den Eintritts-Port (9a) in den Entlüftungskanal (9) eintreten kann.

4. Medikamentinhalator (1) nach einem der vorhergehenden Ansprüche, **dadurch gekennzeichnet, dass** das bewegliche Element (10) eine Kugel ist.

5. Medikamentinhalator (1) nach Anspruch 1, **dadurch gekennzeichnet, dass**:
- der Einlass (20a) der entfernbaren Kassette (20) und der Eintritts-Port (9a) des Entlüftungskanals (9) in Fluidverbindung sind, und
- der Auslass (20b) der entfernbaren Kassette (20) entweder den Entlüftungs-Port (9b) darstellt oder dem Entlüftungs-Port (9b) zugewandt und mit diesem in Fluidverbindung ist.

6. Medikamentinhalator (1) nach einem der vorhergehenden Ansprüche, **dadurch gekennzeichnet, dass** die obere Abdeckung (5) Folgendes umfasst:
- einen Hülsenabschnitt (5a), der eine Hülse um ein oberes Ende (2b) des Gehäuses (2) bildet, wenn die obere Abdeckung (5) an dem Gehäuse (2) angebracht ist,
- einen Dachabschnitt (5b), der an dem Hülsenabschnitt (5a) befestigt ist und den Auslass (7) der oberen Abdeckung (5) umfasst.

7. Medikamentinhalator (1) nach einem der vorhergehenden Ansprüche, **dadurch gekennzeichnet, dass** die innere Kammer (6) durch die inneren Wände des Hülsenabschnitts (5a) und des Dachabschnitts (5b) der oberen Abdeckung (5) begrenzt ist und eine äußere Endwand (2c) des Gehäuses (2), vorzugsweise die äußere Endwand (2c) des Gehäuses (2), die Bodenwand (6a) der inneren Kammer (6) bildet.

8. Medikamentinhalator (1) nach Anspruch 6, **dadurch gekennzeichnet, dass** der Hülsenabschnitt (5a) der oberen Abdeckung (5) ein Fenster (55) umfasst, das dem Entlüftungs-Port (9b; 20b) des Entlüftungskanals (9) zugewandt und mit diesem in Fluidverbindung ist, wenn die obere Abdeckung (5) an dem Gehäuse (2) angebracht ist.

9. Medikamentinhalator (1) nach einem der vorhergehenden Ansprüche, **dadurch gekennzeichnet, dass** das Einwegeventil (10) der Austrittsöffnung (4) des Gehäuses (2) zugewandt ist und/oder einen ringförmigen Umfangsrand (84) umfasst, der zwischen der oberen Kappe (5) und dem Gehäuse (2) angeordnet und eingequetscht ist, wodurch eine Gasdichtung gebildet wird.

10. Medikamentinhalator (1) nach einem der vorhergehenden Ansprüche, **dadurch gekennzeichnet, dass** der Entlüftungskanal (9) eine längliche, bogenförmige Form hat.

11. Medikamentinhalator (1) nach einem der vorhergehenden Ansprüche, **dadurch gekennzeichnet, dass** das bewegliche Element (10) einen Durchmesser von weniger als 10 mm, vorzugsweise von ungefähr 5 mm, hat.

12. Medikamentinhalator (1) nach einem der vorhergehenden Ansprüche, **dadurch gekennzeichnet, dass** das bewegliche Element (10) aus Polymermaterial, vorzugsweise Polyamid, hergestellt ist.

13. Medikamentinhalator (1) nach einem der vorhergehenden Ansprüche, **dadurch gekennzeichnet, dass** das Gehäuse (2) ferner eine untere Abdeckung (15) umfasst, die einen unteren Einlass (16) umfasst, der so ausgestaltet ist, dass eine ADD-Vorrichtung (ADD - Aerosol Drug Delivery - Aerosolmedikamentenverabreichung) darin eingesteckt werden kann.

14. Medikamentinhalator (1) nach einem der vorhergehenden Ansprüche, **dadurch gekennzeichnet, dass** das bewegliche Element (10) ein Gewicht von weniger als 1 g, vorzugsweise weniger als 0,5 g, hat.

15. Aerosoltherapieanordnung, umfassend einen Medikamentinhalator (1) nach einem der vorhergehenden Ansprüche und eine ADD-Vorrichtung, die in den unteren Einlass (16) der unteren Abdeckung (15) des Medikamentinhalators (1) eingesteckt ist.

## Revendications

1. Inhalateur de médicament (1) pour administrer une thérapie gazeuse à un patient, comprenant un boîtier (2), un couvercle supérieur (5) fixé au boîtier (2) et un compartiment intérieur (6) agencé entre ledit boîtier (2) et ledit couvercle supérieur (5), et
- le boîtier (2) comprenant une chambre creuse (3) avec un orifice de sortie (4), et le couvercle supérieur (5) comprenant une sortie (7), ledit orifice de sortie (4) de la chambre creuse (3) et ladite sortie (7) du couvercle supérieur (5) étant en communication fluidique avec le compartiment intérieur (6),
- un canal d'évacuation(9) comprenant un orifice d'entrée (9a) et un orifice (9b), étant en communication fluidique avec le compartiment intérieur (6) par l'intermédiaire de l'orifice d'entrée (9a) et en outre avec l'atmosphère par l'intermédiaire de l'orifice d'évacuation (9b), et
- une valve unidirectionnelle (8) étant agencée dans le compartiment intérieur (6) et configurée pour permettre au gaz de circuler :
i) de la chambre creuse (3) du boîtier (2) à la sortie (7) du couvercle supérieur (5), lorsque le patient inhale du gaz, et
ii) de la sortie (7) du couvercle supérieur (5) au canal d'évacuation (9), lorsque le patient expire du gaz,
le canal d'évacuation (9) comprenant un élément mobile (10) qui se déplace dans ledit canal d'évacuation (9) dans la direction de l'orifice d'évacuation (9b), ledit élément mobile (10) étant retenu dans la zone de l'orifice d'évacuation (9b) et visible de l'extérieur à travers ledit orifice d'évacuation (9b), lorsque le patient expire du gaz par la sortie (7) du couvercle supérieur (5), fournissant ainsi un système de rétroaction visuel permettant de savoir immédiatement de l'extérieur si ledit patient respire du gaz,
**caractérisé en ce que**
- l'élément mobile (10) a une forme sphérique ou cylindrique,
- au moins une partie du canal d'évacuation (9) et l'élément mobile (10) sont agencés dans une cassette amovible (20) ayant une forme arquée allongée, et
- la cassette amovible (20) comprend en outre un passage intérieur avec une entrée (20a) et une sortie (20b), ledit passage intérieur formant au moins une partie du canal d'évacuation (9).

2. Inhalateur de médicament (1) selon la revendication précédente, **caractérisé en ce que** la valve unidirectionnelle (8) est une valve en bec de canard, de préférence au moins partiellement flexible.

3. Inhalateur de médicament (1) selon l'une quelconque des revendications précédentes, **caractérisé en ce que** la valve unidirectionnelle (8) comprend une jupe périphérique flexible (84) qui fléchit vers le boîtier (2), lorsque le patient expire du gaz à travers la sortie (7) du couvercle supérieur (5), permettant ainsi au gaz expiré d'entrer dans le canal d'évacuation (9) via l'orifice d'entrée (9a).

4. Inhalateur de médicament (1) selon l'une quelconque des revendications précédentes, **caractérisé en ce que** l'élément mobile (10) est une balle.

5. Inhalateur de médicament (1) selon la revendication 1, **caractérisé en ce que** :
- l'entrée (20a) de la cassette amovible (20) et l'orifice d'entrée (9a) du canal d'évacuation (9) sont en communication fluidique, et
- la sortie (20b) de la cassette amovible (20) constitue l'orifice d'évacuation (9b), ou fait face à l'orifice d'évacuation (9b) et est en communication fluidique avec celui-ci.

6. Inhalateur de médicament (1) selon l'une quelconque des revendications précédentes, **caractérisé en ce que** le couvercle supérieur (5) comprend :
- une partie manchon (5a) formant un manchon autour d'une extrémité supérieure (2b) du boîtier (2), lorsque le couvercle supérieur (5) est fixé au boîtier (2),
- une partie de toit (5b) fixée à la partie de manchon (5a), et comprenant la sortie (7) du couvercle supérieur (5).

7. Inhalateur de médicament (1) selon l'une quelconque des revendications précédentes, **caractérisé en ce que** la chambre intérieure (6) est délimitée par les parois intérieures de la partie manchon (5a) et de la partie de toit (5b) du couvercle supérieur (5), et une paroi d'extrémité extérieure (2c) du boîtier (2), de préférence la paroi d'extrémité extérieure (2c) du boîtier (2) forme la paroi inférieure (6a) de la chambre intérieure (6).

8. Inhalateur de médicament (1) selon la revendication 6, **caractérisé en ce que** la partie de manchon (5a) du couvercle supérieur (5) comprend une fenêtre (55) faisant face et en communication fluidique avec l'orifice d'évacuation (9b ; 20b) du canal d'évacuation (9), lorsque le couvercle supérieur (5) est fixé au boîtier (2).

9. Inhalateur de médicament (1) selon l'une quelconque des revendications précédentes, **caractérisé en ce que** la valve unidirectionnelle (10) faisant face à l'orifice de sortie (4) du boîtier (2) et/ou comprenant une bordure annulaire périphérique (84) est prise en sandwich et écrasée entre le couvercle supérieur (5) et le boîtier (2), formant ainsi un joint d'étanchéité aux gaz.

10. Inhalateur de médicament (1) selon l'une quelconque des revendications précédentes, **caractérisé en ce que** le canal d'évacuation (9) présente une forme allongée en arc de cercle.

11. Inhalateur de médicament (1) selon l'une quelconque des revendications précédentes, **caractérisé en ce que** l'élément mobile (10) présente un diamètre inférieur à 10 mm, de préférence d'environ 5 mm.

12. Inhalateur de médicament (1) selon l'une quelconque des revendications précédentes, **caractérisé en ce que** l'élément mobile (10) est réalisé en matériau polymère, de préférence en polyamide.

13. Inhalateur de médicament (1) selon l'une quelconque des revendications précédentes, **caractérisé en ce que** le boîtier (2) comprend en outre un couvercle inférieur (15) comprenant une entrée inférieure (16) configurée pour y brancher un dispositif d'administration de médicaments en aérosol (ADD).

14. Inhalateur de médicament (1) selon l'une quelconque des revendications précédentes, **caractérisé en ce que** l'élément mobile (10) a un poids inférieur à 1 g, de préférence inférieur à 0,5 g.

15. Ensemble d'aérosolthérapie comprenant un inhalateur de médicament (1) selon l'une quelconque des revendications précédentes et un dispositif d'administration de médicament en aérosol (ADD) branché dans l'entrée inférieure (16) du couvercle inférieur (15) dudit inhalateur de médicament (1).
